## Europäisches Patentamt

## European Patent Office

## Office européen des brevets

(19)

(11) Veröffentlichungsnummer: **0 289 940**
**A2**

# EUROPÄISCHE PATENTANMELDUNG

(21) Anmeldenummer: **88106809.2**

(22) Anmeldetag: **28.04.88**

(51) Int. Cl.4 **A61F 9/02 , A62B 18/08**

(30) Priorität: **02.05.87 DE 3714731**

(43) Veröffentlichungstag der Anmeldung:
**09.11.88 Patentblatt 88/45**

(34) Benannte Vertragsstaaten:
**AT BE CH DE FR GB IT LI LU NL SE**

(71) Anmelder: **Drägerwerk Aktiengesellschaft**
**Moislinger Allee 53-55**
**D-2400 Lübeck 1(DE)**

(72) Erfinder: **Schlobohm, Joachim**
**Salinenstrasse 7**
**D-2400 Lübeck(DE)**

(54) **Schutzmaske mit zur Benutzung optischer Hilfsmittel geeigneten Augenfenstern.**

(57) Eine Schutzmaske mit zur Benutzung optischer Hilfsmittel geeigneten Augenfenstern, deren Rahmen gegenüber dem Maskenkörper in eine vorgegebene Neigung gebracht sind und deren Durchsichtbereich im wesentlichen in einer gemeinsamen Ebene liegt, soll derart verbessert werden, daß das Gesichtsfeld und die Position der Augenfenster den Einsatzbedingungen angepaßt werden können. Insbesondere sollen unter normalen Einsatzbedingungen keine Gesichtsfeldeinschränkungen auftreten. Die erforderliche Bauform der Augenfenster soll ein Zusammenfalten des Maskenkörpers zum Zwecke einer raumsparenden Lagerung oder eines Transportes nicht verhindern. Dazu sind die Rahmen (4) im Schläfenbereich des Maskenkörpers (1) von einem aus dem Maskenkörper (1) herausragenden Seitenwulst (5) aufgenommen, welcher zum Maskenkörper (1) hin eindrückbar ist.

Fig. 1

EP 0 289 940 A2

## Schutzmaske mit zur Benutzung optischer Hilfsmittel geeigneten Augenfenstern

Die Erfindung betrifft eine Schutzmaske mit zur Benutzung optischer Hilfsmittel geeigneten Augenfenstern, deren Rahmen gegenüber dem Maskenkörper in eine vorgegebene Neigung gebracht sind und deren Durchsichtbereich im wesentlichen in einer gemeinsamen Ebene liegt.

Eine derartige Schutzmaske ist aus der DE-PS 472 597 bekanntgeworden.

Wenn ein Schutzmaskenträger optische Hilfsmittel, wie z. B. Ferngläser, benutzen muß, so sind die üblicherweise dem Verlauf der Gesichtskontur angepaßten Augenfenster dazu nicht geeignet, weil die Okulare der Ferngläser in einer gemeinsamen Ebene liegen. Ein lichtdichtes und möglichst kurzes Heranführen der Okulare an die Augen des Maskenträgers ist nicht möglich. Bei der bekannten Schutzmaske wird dazu das Augenfenster in eine trichterförmige Vertiefung der Maskenwand eingelassen, um möglichst nahe an die Augenpupillen des Maskenträgers herangeführt werden zu können. Bei dieser bekannten Anordnung der Augenfenster ist es von Nachteil, daß das Gesichtsfeld des Maskenträgers unvertretbar eingeengt ist, sofern er die optischen Hilfsmittel, wie z. B. ein Fernglas, nicht mehr benutzt. Durch die tieferliegenden Sichtfenster entstehen im Nasen-und Schläfenbereich der Schutzmaske Vertiefungen, die den Blickwinkel des Maskenträgers begrenzen.

Andere bekannte Ausformungen der Sichtfenster, wie sie z. B. in der EP-A 106 447 gezeigt sind, besitzen zwar zur Erzielung eines möglichst großen Gesichtsfeldes eine gewölbte, den Gesichtskonturen angepaßte Neigung, verhindern aber somit ein ebenes Anliegen der optischen Hilfsmittel, beispielsweise der Okulare eines Fernglases.

Bei beiden bekannten Schutzmasken ist es fernerhin nicht möglich, die Maskenkörper zum Zwecke einer raumsparenden Lagerung oder eines raumsparenden Transportes zusammenzufalten, beispielsweise in einem an der Person getragenen Maskenbehälter, weil die eingezogenen Umrandungen der Augenfenster unmittelbar neben dem Nasenbereich der Schutzmaske ein derartiges Zusammenlegen des Maskenkörpers verhindern.

Der vorliegenden Erfindung liegt somit die Aufgabe zugrunde, eine Schutzmaske der genannten Art so zu verbessern, daß das Gesichtsfeld und die Position der Augenfenster den Einsatzbedingungen angepaßt werden können. Insbesondere sollen Gesichtsfeldeinschränkungen lediglich bei Benutzung von optischen Hilfsmitteln wirksam werden können. Die erforderliche Bauform der Augenfenster soll ein Zusammenfalten des Maskenkörpers zum Zwecke einer raumsparenden Lagerung oder eines Transportes nicht verhindern.

Die Lösung der Aufgabe erfolgt dadurch, daß die Rahmen im Schläfenbereich des Maskenkörpers von einem aus dem Maskenkörper herausragenden Seitenwulst aufgenommen sind, welcher zum Maskenkörper hin eindrückbar ist.

Die Vorteile der Erfindung liegen im wesentlichen darin, daß zur Benutzung optischer Hilfsmittel die Augenfenster, ausgehend von dem herkömmlich geformten Nasenbereich der Schutzmaske, im wesentlichen lotrecht zur Mittelebene des Maskenkörpers bis zum Schläfenbereich verlaufen und dort von dem aus dem Maskenkörper herausragenden Seitenwulst aufgenommen sind. Werden die optischen Hilfsmittel nicht mehr benötigt, kann der Seitenwulst eingedrückt werden, so daß die Augenfenster in einen spitzen Winkel zur Mittelebene des Maskenkörpers gebracht werden. Dadurch ist eine erhebliche Erweiterung des Sichtbereichs, wie er bei herkömmlichen Schutzmasken geläufig ist, erreicht worden. Da die Augenfenster im Nasenbereich des Maskenkörpers keinerlei Einbuchtungen oder Vertiefungen besitzen, kann der Maskenkörper zu einer flachen Packung zusammengedrückt werden, wobei die Seitenwulste ebenfalls an den zusammengedrückten Maskenkörper zusammengepreßt werden können. Damit ist ein platzsparendes Transportieren und Lagern möglich.

Der Maskenkörper kann so ausgebildet sein, daß bei eingedrücktem Seitenwulst der Augenfensterrahmen durch eine vorspringende Nase in dieser Position gehalten ist. Zweckmäßigerweise kann auch eine am Maskenkörper angreifende Spannvorrichtung vorgesehen sein, welche beispielsweise aus einer mit dem Maskenkörper verbundenen Lasche besteht, die nach dem Eindrücken des Seitenwulstes mittels einer Öse über einen am Seitenwulst befestigten Noppen eingehakt werden kann.

Vorteilhafterweise ist der Seitenwulst einstückig mit dem Maskenkörper geformt und bildet so eine elastische Ausbuchtung im Bereich der Augenfenster.

Ein Ausführungsbeispiel der Erfindung wird anhand der schematischen Zeichnung dargestellt und im folgenden näher erläutert.

Es zeigen:

Fig. 1 die Teilansicht einer Schutzmaske im Augenfensterbereich

Fig. 2 die Draufsicht auf die Schutzmaske im Augenfensterbereich.

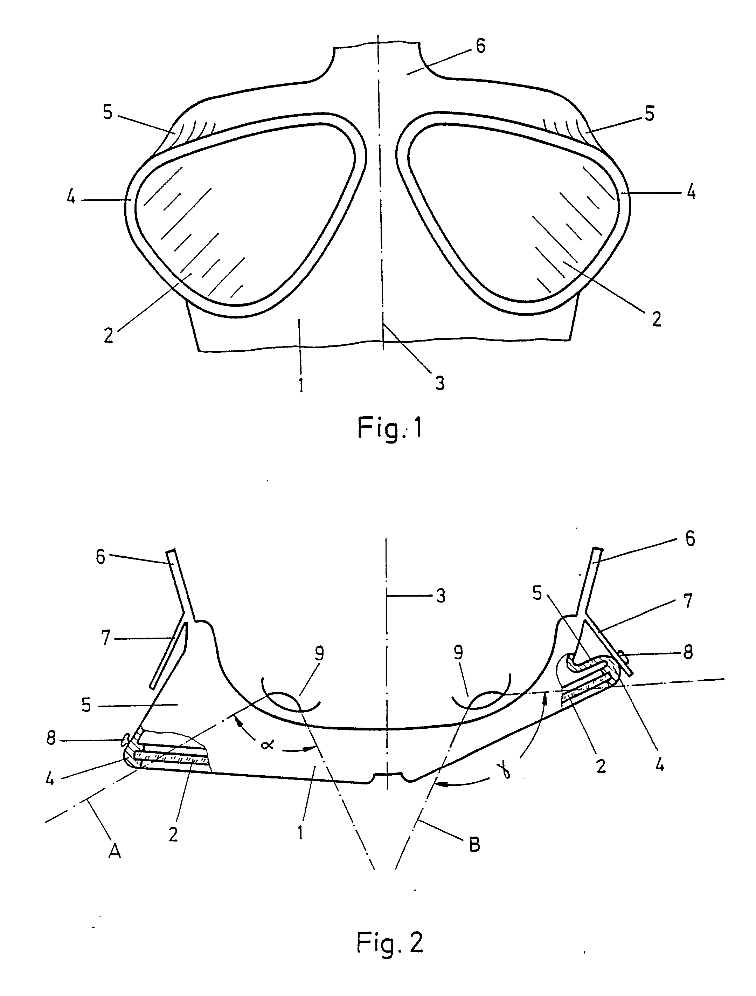

Der in Figur 1 dargestellte Maskenkörper (1) zeigt lediglich den Bereich um die Augenfenster (2), welche symmetrisch zur strichpunktiert dargestellten Mittellinie (3) angeordnet sind. Die Augenfenster (2) sind in einem Rahmen (4) gehalten, der sich von der Mittellinie (3) zum Schläfenbereich des Maskenkörper in einer ovalähnlichen Form erstreckt. Die Rahmen (4) sind im Schläfenbereich des Maskenkörpers (1) durch einen Seitenwulst (5) getragen, der sich von der äußeren Kontur des Maskenkörpers (1) derart abhebt, daß sich die Augenfenster (2) annähernd in einer Ebene befinden. Im Stirnbereich des Maskenkörpers (1) in Höhe der Mittellinie (3) ist ein Ansatz (6) für die weiter nicht dargestellte Maskenbänderung vorgesehen.

Der Maskenkörper (1) ist in Figur 2 in einer teilgeschnittenen Draufsicht auf den Bereich der Augenfenster (2) gezeigt. Dabei ist der links von der Mittellinie (3) befindliche Teil mit dem aus dem Maskenkörper (1) herausragenden Seitenwulst (5) dargestellt. Der rechts von der Mittellinie (3) gezeigte Maskenteil stellt den Wulst (5) in der zum Maskenkörper (1) eingedrückten Position dar. Der die Augenfenster (2) aufnehmende Teil des Rahmens (4) ist in jeweiligen Durchbrechungen des Seitenwulstes (5) gezeigt. Die Einspannung des eingedrückten Seitenwulstes (5) erfolgt mit einer Lasche (7), die einstückig mit dem Maskenkörper (1) verbunden ist und die an ihrem freien Ende eine Öse aufweist, mit welcher die Lasche (7) über einen Noppen (8) am Rahmen (4) befestigt ist. Der Sichtbereich für die Augen (9) des Maskenträgers ist bei aus dem Maskenkörper (1) herausragendem Augenfenster (2) entsprechend dem mit der strichpunktierten Linie A dargestellten Winkel alpha enger, als der bei eingedrücktem Seitenwulst (5), entsprechend der strichpunktierten Linie B, dessen Sichtwinkel gamma deutlich größer und mit den üblichen, wulstfreien Augenfenstern bekannter Schutzmasken vergleichbar ist.

## Ansprüche

1. Schutzmaske mit zur Benutzung optischer Hilfsmittel geeigneten Augenfenstern, deren Rahmen gegenüber dem Maskenkörper in eine vorgegebene Neigung gebracht sind und deren Durchsichtbereich im wesentlichen in einer gemeinsamen Ebene liegt, dadurch gekennzeichnet, daß die Rahmen (4) im Schläfenbereich des Maskenkörpers (1) von einem aus dem Maskenkörper (1) herausragenden Seitenwulst (5) aufgenommen sind, welcher zum Maskenkörper (1) hin eindrückbar ist.

2. Schutzmaske nach Anspruch 1, dadurch gekennzeichnet, daß der Seitenwulst (5) in eingedrücktem Zustand mit einer am Maskenkörper (1) angreifenden Spannvorrichtung (7, 8) gehalten ist.

3. Schutzmaske nach Anspruch 1 oder 2, dadurch gekennzeichnet, daß der Seitenwulst (5) einstückig aus dem Maskenkörper (1) geformt ist.

Fig. 1

Fig. 2